# EUROPÄISCHE PATENTSCHRIFT

(11) **EP 0 457 127 B1**
(45) Veröffentlichungstag und Bekanntmachung des Hinweises auf die Patenterteilung: **21.08.1996**
(21) Anmeldenummer: 91107177.7
(22) Anmeldetag: 03.05.1991
(51) Int. Cl.: A61L 31/00, A61F 6/04

(54) **Spermicides Beschichtungsmittel**
Spermicidal coating
Matériau de revêtement spermicide

(30) Priorität: 15.05.1990 DE 4015543
(43) Veröffentlichungstag der Anmeldung: 21.11.1991
(73) Patentinhaber: BAYER AG, 51368 Leverkusen (DE)
(72) Erfinder: Marquardt, Gerwig, Dr., W-5068 Odenthal (DE); Preiss, Peter, D. I., W-5600 Wuppertal 1 (DE)

(56) Entgegenhaltungen:
- EP-A- 0 328 421
- WO-A-89/04647
- DATABASE WPIL Section Ch, Week 8742, Derwent Publications Ltd., London, GB; Class A96, AN 87-294036

## Beschreibung

Die vorliegende Erfindung betrifft Beschichtungsmittel auf Siliconbasis für die Behandlung von Latexmaterialien, insbesondere von Kondomen und ähnlichen Artikeln mit dem Vorteil einer Löslichkeit für spermicide Wirkstoffe.

Latexmaterialien werden in vielfältiger Weise, u.a. auch im sanitären Bereich, z.B. als Schutzhandschuhe, Abdeckungen, Schutz für Bandagen, insbesondere aber für Kondome eingesetzt.

Kondomen werden angesichts der zunehmenden Gefahr von AIDS-Erkrankungen als Schutz- und Verhütungsmittel vermehrt Aufmerksamkeit geschenkt.

Auf der Basis wissenschaftlicher Untersuchungen bietet die Verwendung eines Kondoms als genitales HIV-Prophylaktikum den derzeit bestmöglichen Schutz (vgl. Knut O.K. Hoffmann: Wie sicher ist das Kondom?, ZFA 7/1988).

Die industrielle Fertigung des Kondoms seit etwa 65 Jahren geht auf Julius Fromm zurück und hat heute in bezug auf die Materialqualität dieser Artikel einen hohen Standard erreicht. Für seine Herstellung wird fast ausschließlich Latex verwendet, welches sich in erster Linie durch enorme Reißfestigkeit, Dehnungsfähigkeit, und Dichtigkeit auszeichnet.

Darüber hinaus lassen sich Kondome auf Basis von Latex in äußerst dünnen Schichtstärken von ca. 0,08 mm problemlos produzieren. Aufgrund der relativ schlechten Gleitfähigikeit von Latex wird nach der Herstellung dieser Artikel meist noch ein Gleitmittel appliziert, um Probleme bei der Benutzung zu minimieren.

Während früher üblicherweise Festsubstanzen, wie Talkum Anwendung fanden, sind heute meist ölartige Produkte zur Gleitverbesserung im Einsatz.

In der Patentliteratur sind eine Vielzahl unterschiedlicher Gleitmittel oder Zubereitungen beschrieben, wobei hauptsächlich den Glycolen und klassischen Methylsiliconölen besondere Bedeutung beigemessen wird.

So wird beispielsweise in der japanischen Patentanmeldung JP 52 138 397 die Verwendung von feinen Glaskügelchen in Kombination mit Siliconöl, verschiedenen Glycolen, Glycerin oder modifizierten Alkoholen beschrieben, während die JP 70 006 119 ein System aus Siliconöl und Pteridophytasporen zum Inhalt hat.

Desweiteren werden in JP 59 141 942 Siliconöle oder wasserlösliche Polymere zur Gleitverbesserung genannt.

Speziell im Hinblick auf eine spermicide Ausrüstung der Gleitmittel sind Kombinationen von modifizierten Alkoholen, Algenaten, Propylenglycol, Gelatine, Kochsalz und Sacchariden (JP 74 019 799) beschrieben sowie Polyethylenglycol in Kombination mit Siliconölen (JP 62 164 460).

Aus der Vielzahl der verwendbaren Systeme haben sich Methylsiliconöle (PDMS) mittlerer Viskositäten als bevorzugte Feuchtbeschichtungsmittel durchsetzen können, da sie wegen ihrer guten Schleimhautverträglichkeit, Gleiteigenschaften und Verträglichkeit mit Latex Vorteile bieten gegenüber Glycolen oder anderen hydrophilen Systemen. Darüber hinaus sind PDMS Substanzen, die nicht bevorzugt von Bakterien befallen werden und bei sachgemäßer Herstellung als extrem keimarm angesehen werden können.

Wegen der zunehmenden Gefahr einer möglichen Ansteckung durch den HIV-Virus beim Geschlechtsverkehr kommen Beschichtungsmitteln, die in vitro die Aktivität der reversen Transkriptase bei der HIV-Replikation hemmen, eine größere Bedeutung zu als bisher. Durch Untersuchungen von Hicks (vgl. "Inactivation of HIV-III/LAV-infected cultures of normal human lymphocytes by Nonoxinol-9 in vitro". Lancet II, 1422-1423, 1985) ist bekannt, daß bei spermiciden Substanzen, z.B. vom Typ Nonoxinol-9, einer nicht-ionischen, oberflächenaktiven Substanz, eine derartige Wirksamkeit zu beobachten ist.

Es wäre nun wünschenswert, wenn derartige Sustanzen in den bisher zur Anwendung kommenden Siliconölen löslich wären, was aber wegen des unterschiedlichen chemischen Aufbaus beider Substanzklassen leider nicht der Fall ist.

Nonoxinol-9 besitzt die chemische Bezeichnung 26-(4-Nonylphenoxy)-3, 6, 9, 12, 15, 18, 21, 24-octaoxahexacosan-1-ol und ist somit im weitesten Sinne ein modifizierter Polyether, der damit verträglich bzw. löslich ist in Glycolen, Polyglycolen oder Polyethern.

Für die spermicide Ausrüstung von Kondomen wird deshalb als Trägermaterial für Nonoxinol-9 fast ausschließlich Glycol, Polyglycol oder ein glycolähnliches System verwendet. Diese hydrophilen Substanzen zeigen im allgemeinen eine relativ schlechte Benetzbarkeit der Kondomoberfläche und eine weniger gut ausgeprägte Verträglichkeit mit Latex. Darüber hinaus vermitteln sie dem Kondom eine im Vergleich zu PDMS höhere Klebrigkeit.

Aufgabe der Erfindung war es nun, Siliconsysteme für die Ausrüstung von Kondomen zu verwenden, die Löslichkeitseigenschaften für Wirkstoffe auf Nonoxinol-Basis, bevorzugt Nonoxinol-9 besitzen, um so die Herstellung lagerstabiler Beschichtungsmittel mit spermicider Wirksamkeit zu ermöglichen.

Desweiteren sollten diese Beschichtungsmittel hinsichtlich Gleitverhalten, Klebrigkeit und Schleimhautverträglichkeit dem Eigerschaftsbild der PDMS ziemlich ähnlich sein.

Gegenstand der vorliegenden Erfindung sind nun spermicide Beschichtungsmittel zur Beschichtung von Latexmaterialien enthaltend einen oder mehrere spermicid wirkende Stoffe, insbesondere auf Nonoxinol-9-Basis, sowie eine oder mehrere Organopolysiloxan-Verbindungen, in denen die Wirkstoffe löslich sind.

Diese Aufgabe konnte insbesondere gelöst werden durch Verwendung hydroxyalkylgruppenhaltiger Siloxane mit folgendem Aufbau: 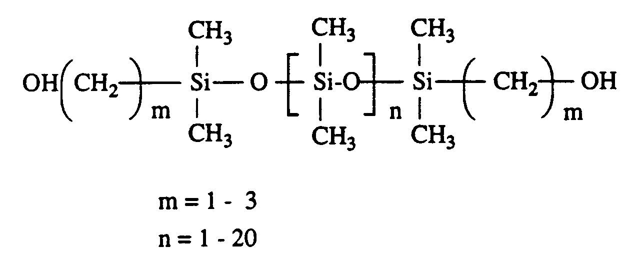
zum anderen auch durch die Substanzklasse der Polyether-Polysiloxan-Copolymere linearer oder verzweigter Struktur. Der grundsätzliche Aufbau dieser Verbindungen kann durch die folgenden drei Formelbilder wiedergegeben werden.

### Typ 1

seitenständige Anbindung des Polyetherrestes am linear aufgebauten Siloxan.

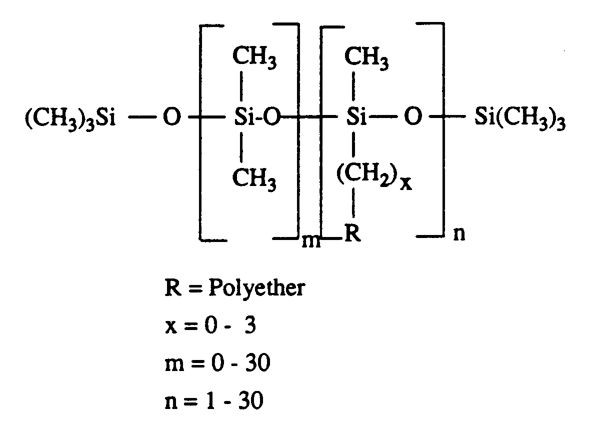

### Typ 2

endständige Anbindung des Polyetherrestes am linear aufgebauten Siloxan.

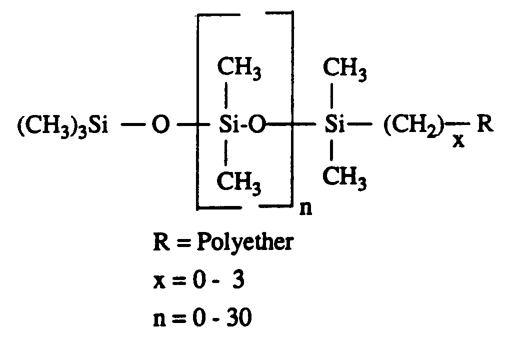

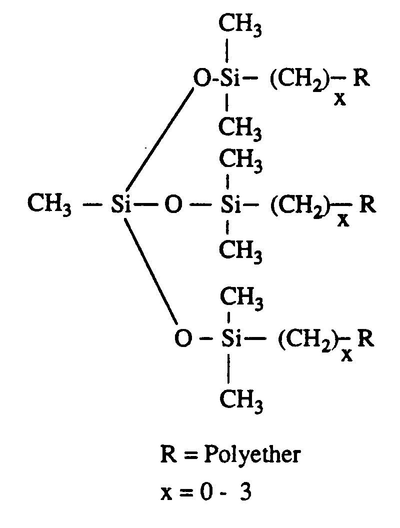

Im Prinzip können auch noch andere Polyether-Polysiloxan-Copolymere mit modifizierten Strukturen für die Zubereitung der erfindungsgemäßen Beschichtungsmittel verwendet werden.

Hydroxyalkylgruppenhaltige Siloxane lassen sich beispielsweise herstellen aus Chlormethylchlorsilan, Umsetzung zum Dihydroxymethyltetramethyldisiloxan und Äquilibrierung mit Octamethylcyclotetrasiloxan.

Ausgangsprodukte für die Fertigung von Polyether-Polysiloxan-Copolymeren sind Polysiloxane, die innerhalb der Polymerkette (seitenständig) oder an ihren Enden (endständig) reaktive Gruppen wie ≡SiCl, ≡SiOR oder ≡SiH enthalten. Diese Vorpolymere werden gewonnen entweder durch Hydrolyse von Methylchlorsilanen oder durch Copolymerisation von cyclischen oder linearen Polymethylsiloxanen mit funktionellen Siloxanen. Nachfolgend schließt sich bei beiden Verfahren eine Äquilibrierungsreaktion an, in der mit sauren Katalysatoren durch Aufbrechen und Neuknüpfen der Si-O-Si-Bindungen die Dimethylsiloxy- und die funktionellen Siloxygruppen in das statistische Gleichgewicht gebracht werden. Die Umsetzung dieser reaktiven Siloxane mit Polyethern führt dann zu Polyether-Polysiloxan-Copolymeren.

Beide beschriebenen Substanzklassen sind seit langem bekannt (z.B. aus Walter Noll: Chemie und Technologie der Silicone, 2. Auflage, 1968, Verlag Chemie GmbH, Weinheim/Bergstraße) und werden je nach Aufbau verwendet als Oberflächenveredelungsprodukte für Natur- und Kunstleder, als Schaumstabilisatoren bei der PU-Verarbeitung, als Tenside und Antistatika sowie als hautverträgliche Komponenten in verschiedenen dermatologischen und kosmetischen Rezepturen. Im Rahmen ihrer vielfältigen Anwendungsmöglichkeiten sind bislang keinerlei negativen dermatologischen Effekte bei ihrer Verarbeitung festgestellt worden.

Anhand der nachfolgenden Beispiele soll nun die Wirksamkeit der erfindungsgemäßen Substanzklassen näher verdeutlicht werden.

### Beispiel 1

0,3 g einer Lösung, bestehend aus 6 Gew.-Teilen Nonoxinol-9 und 94 Gew.-Teilen α,γ-Hydroxymethyldimethylsilylpolydimethylsiloxan mit 12 Dimethylsilyloxyeinheiten wurden auf die Kuppe eines aufgerollten Kondoms aus Latex aufgetropft. Der so behandelte Artikel wurde anschließend eingesiegelt und einige Tage bei Raumtemperatur gelagert. Während dieser Zeit kam es dann aufgrund der Spreiteigenschaften des Silicons zu einer vollständige Belegung der Kondomoberfläche mit dem den spermiciden Wirkstoff enthaltenden Beschichtungsmittel. Bei der nachfolgenden haptischen Prüfung des behandelten Artikels zeigte sich eine deutliche Verbesserung der Gleiteigenschaften im Vergleich mit unbehandelter Ware, verbunden mit einer nur sehr gering ausgeprägten Klebrigkeit. Eine Überprüfung der mechanischen Eigenschaften von Kondomen, die 14 Tage bei 50°C in dem verwendeten Beschichtungsmittel gelagert wurden, ergab keine Hinweise auf eine Veränderung der Latexqualität auch nach längerem Kontakt mit der Siliconpräparation.

### Beispiel 2

0,5 g einer Lösung, bestehend aus 6 Gew.-Teilen Nonoxinol-9, 20 Gew.-Teilen Decamethylcyclopentasiloxan und 74 Gew.-Teilen eines verzweigten Polyether-Polysiloxan-Copolymers vom Typ 3 mit x = 1 und einem 75 %igen Polyetheranteil, bestehend aus Ethoxy- und Propoxyeinheiten, wurden gemäß Beispiel 1 auf die Oberfläche eines Kondoms appliziert und geprüft. Auch hier zeigte sich bei den haptischen Tests ein günstiges Gleitverhalten und eine geringe Klebrigkeit. Eine mechanische Veränderung der Latexqualität nach Lagerprüfing konnte nicht festgestellt werden.

### Beispiel 3

0,4 g einer Lösung, bestehend aus 6 Gew.-Teilen Nonoxinol-9 und 94 Gew.-Teilen eines linear aufgebauten Polyether-Polysiloxan-Copolymers vom Typ 1 mit m = 10, n = 1, x = 3 und einem 60 %igen, seitenständig angebundenen Polyetheranteil, bestehend aus Ethoxy- und Propoxyeinheiten, wurden gemäß Beispiel 1 auf die Oberfläche eines Kondoms aufgebracht, und der so behandelte Artikel anschließend geprüft. Es zeigte sich bei den haptischen Tests ein günstiges Gleitverhalten und eine sehr geringe Klebrigkeit. Eine mechanische Veränderung der Latexqualität nach Lagerprüfung war nicht festzustellen.

### Beispiel 4

0,4 g einer Lösung, bestehend aus 6 Gew.-Teilen Nonoxinol-12 und 94 Gew.-Teilen eines linear aufgebauten Polyether-Polysiloxan-Copolymers vom Typ 1 mit m = 10, n = 1, x = 3 und einem 60 %igen, seitenständig gebundenen Polyetheranteil, bestehend aus Ethoxy- und Propoxyeinheiten, wurden gemäß Beispiel 1 auf die Oberfläche eines Kondoms aufgebracht, und der so behandelte Artikel anschließend geprüft. Es zeigte sich bei den haptischen Tests ein günstiges Gleitverhalten und eine sehr geringe Klebrigkeit. Eine mechanische Veränderung der Latexqualität nach Lagerprüfung war nicht festzustellen.

### Beispiel 5

0,3 g einer Lösung, bestehend aus 6 Gew.-Teilen Nonoxinol-9 und 94 Gew.-Teilen eines linear aufgebauten Polyether-Polysiloxan-Copolymers vom Typ 2 mit n = 12, x = 3 und einem 50 %igen, endständig angebundenen Polyetheranteil, bestehend aus Ethoxy- und Propoxyeinheiten, wurden gemäß Beispiel 1 auf die Oberfläche eines Kondoms aufgebracht und der so behandelte Artikel anschließend geprüft. Auch hier war bei der haptischen Prüfung ein sehr gutes Gleitverhalten, verbunden mit geringer Klebrigkeit, zu beobachten. Eine mechanische Veränderung der Latexqualität nach Lagerprüfung konnte wie bei den vorangegangenen Beispielen nicht festgestelt werden.

## Patentansprüche

1. Spermicides Beschichtungsmittel zur Beschichtung von Latexmaterialien enthaltend einen oder mehrere spermicid wirkende Stoffe sowie ein oder mehrere Organopolysiloxanverbindungen, in denen die Wirkstoffe löslich sind.

2. Beschichtungsmittel nach Anspruch 1, dadurch gekennzeichnet, daß es als spermicid wirkender Stoff Nonoxinol-9 enthält.

3. Beschichtungsmittel gemäß einem der Ansprüche 1 oder 2, dadurch gekennzeichnet, daß es als Organopolysiloxanverbindungen solche enthält, die an Si gebundene Hydroxyalkylgruppen oder Polyetherreste aufweisen.

4. Verwendung der Beschichtungsmittel gemäß einem der Ansprüche 1 bis 3 für Kondome.

## Claims

1. A spermicidal coating composition for coating latex materials containing one or more spermicidal agents and one or more organopolysiloxane compounds in which the spermicidal agents are soluble.

2. A coating composition as claimed in claim 1, characterized in that it contains Nonoxinol-9 as the spermicidal agent.

3. A coating composition as claimed in claim 1 or 2, characterized in that it contains organopolysiloxane compounds with hydroxyalkyl groups or polyether functions attached to Si as the organopolysiloxane compounds.

4. The use of the coating compositions claimed in any of claims 1 to 3 for condoms.

## Revendications

1. Produit de revêtement spermicide permettant le revêtement de matériaux latex, comportant un ou plusieurs produits à action spermicide ainsi qu'un ou plusieurs composés organo-polysiloxanes dans lesquels ces produits actifs sont solubles.

2. Produit de revêtement selon la revendication 1, caractérisé en ce qu'il contient du nonoxinol-9 comme produit à action spermicide.

3. Produit de revêtement selon l'une des revendications 1 ou 2, caractérisé en ce qu'il contient comme composés organo-polysiloxanes ceux qui présentent des groupes hydroxyalkyles liés au Si ou des restes de polyéther.

4. Utilisation du produit de revêtement selon l'une des revendications de 1 à 3 pour les condoms.
